# EUROPEAN PATENT SPECIFICATION

(11) **EP 4 025 170 B1**
(45) Date of publication and mention of the grant of the patent: **17.09.2025**
(21) Application number: 20861783.7
(22) Date of filing: 24.08.2020
(51) Int. Cl.: A61H 23/00, B06B 1/10

(54) **PERSONAL USE EXTRACORPOREAL DEVICES AND METHODS OF USE**
EXTRAKORPORALE VORRICHTUNGEN FÜR DEN PERSÖNLICHEN GEBRAUCH UND VERFAHREN ZUR VERWENDUNG
DISPOSITIFS EXTRACORPORELS À USAGE PERSONNEL ET PROCÉDÉS D'UTILISATION

(30) Priority: 02.09.2019 US 201962894913 P; 22.09.2019 US 201962903926 P; 21.01.2020 US 202062964053 P; 16.04.2020 US 202016850885; 16.04.2020 US 202016850878; 16.04.2020 US 202016850890
(43) Date of publication of application: 13.07.2022
(73) Proprietor: Hoffman, Jonathan, Agoura Hills, California 91301 (US)
(72) Inventor: Hoffman, Jonathan, Agoura Hills, California 91301 (US)
(74) Representative: IK-IP LTD
(86) International application number: PCT/US2020/047573
(87) International publication number: WO 2021/045918

(56) References cited:
- EP-B1- 2 095 843
- EP-B1- 2 549 972
- WO-A1-2013/107898
- CN-U- 202 355 560
- DE-U1- 202004 011 323
- US-A- 2 655 921
- US-A- 3 837 335
- US-A- 4 991 568
- US-A1- 2011 245 736
- US-A1- 2014 088 465
- US-B1- 6 770 043
- US-B2- 9 931 151

## Description

### CROSS-REFERENCE TO RELATED APPLICATIONS

The present application claims the benefit of U.S. Provisional Application Serial No. 62/894,913, filed September 2, 2019, entitled "PERSONAL USE EXTRACORPOREAL LOW INTENSITY SHOCK WAVE MECHANICAL TIP AND METHODS OF USE," U.S. Provisional Application Serial No. 62/903,926, filed September 22, 2019, entitled "PERSONAL USE EXTRACORPOREAL LOW INTENSITY SHOCK WAVE DEVICE ENHANCED USER FEATURES AND FUNCTIONS," U.S. Provisional Application Serial No. 62/964,053, filed January 21, 2020, entitled "PERSONAL USE EXTRACORPOREAL LOW INTENSITY SHOCK WAVE DEVICE TIP FORCE DETECTION AND ANNUNCIATION MEANS," U.S. Application Serial No. 16/850,878, filed April 16, 2020, entitled "PERSONAL USE EXTRACORPOREAL LOW INTENSITY ACOUSTIC OR SHOCK WAVE MECHANICAL TIP AND METHODS OF USE," U.S. Application Serial No. 16/850,885, filed April 16, 2020, entitled "PERSONAL USE EXTRACORPOREAL LOW INTENSITY SHOCK WAVE DEVICE ENHANCED USER FEATURES AND FUNCTIONS," and U.S. Application Serial No. 16/850,890, filed April 16, 2020, entitled "PERSONAL USE EXTRACORPOREAL LOW INTENSITY SHOCK WAVE DEVICE TIP FORCE DETECTION AND ANNUNCIATION MEANS AND METHODS FOR USING SAME".

### FIELD OF THE DISCLOSURE

The disclosure relates to non-invasive home use medical devices. More particularly, the present disclosure relates to non-invasive home use medical devices utilizing low intensity acoustic waves for, for example, treating erectile dysfunction or removing cellulite, and for non- invasive home use medical devices utilizing acoustic waves for massage, increasing local blood flow, breaking up plaques in blood vessels, fostering angiogenesis, and cellulite removal.

### BACKGROUND OF THE DISCLOSURE

Acoustic wave treatments and low intensity extracorporeal shock wave treatments are well known in the art and have been widely known and used in the professional medical community for several decades. The treatment methodology has been demonstrated to be effective in treating soft tissue injuries or damage, reducing fatty deposits commonly known as cellulite, and most recently for the treatment of male erectile dysfunction.

Conventional devices are intended to be utilized by a trained, skilled professional applying the treatment to a third-party patient or subject. Such devices require specific knowledge about human anatomy, treatment protocol and regimen, rate of travel of device, pressure to be applied by device, duration of treatment, and frequency of treatment. Further, the controls and displays of such devices are so positioned as to be visible and accessible to a third-party professional treatment specialist, not to a self-user. Further the cost of such devices may be prohibitively high, rendering the purchase or use of such devices beyond the means of the average user.

EP 2 549 972 discloses a device for massaging or treating the muscles of the back and neck of a patient, comprising a housing in which a drive motor drives an annular crank track by means of a gearbox. A lift motion acting on a massage finger is generated by means of a guide unit. The geometric dimensions of the annular crank track determine the constant amplitude or the constant stroke of the massage finger or of the massage head thereof. The massage finger is thus displaced at a constant amplitude, while the frequency is changed in accordance with a controller. US 2 655 921 discloses a vibratory tool for operating bone sets, bone chisels, and bone nail drivers. DE 20 2004 011323 discloses an instrument for applying vibrations to the human body is disclosed, comprising a housing in which an axially movable ram is arranged at the distal end, and a drive device for the ram, for setting the ram in a reciprocating motion, wherein the drive device comprises an axially movable projectile arranged proximally of the ram and an accelerating device for the projectile for accelerating the projectile distally in the direction of the ram, the ram being able to execute a stroke of at least 1 mm. US 9 931 151 discloses an impactor having a housing including an upper portion and a lower portion at least a part of which extends perpendicularly from the upper portion, a ram having a first end positioned in the upper portion and positioned in axial alignment with the lower portion, an opposite end of the ram extending from the lower portion of the housing, at least one drive assembly including a first drive shaft operably coupled to a cam shaft, a cam operably coupled to the cam shaft, a cam operably coupled to the cam shaft, the drive shaft operably connectable to motor, wherein the cam is operable to rotate and positioned to contact the proximal end of the ram upon rotation of the cam, a circuit board comprising circuitry operable to receive instructions from a plurality of switches electrically coupled to the circuit board to operate the impactor, wherein the impactor is operable to deliver low amplitude pulsed strikes to a workpiece at variable frequencies. EP 2 095 843 discloses a device having a striking unit and an impact body, which is made up of sintered ceramic. The ceramic contains 80 percent weight of oxides, carbides or nitrides. The impact body has a cylindrical shape with an entrance surface perpendicular to a cylinder axis and an exit surface perpendicular to the cylinder axis. US 3 837 335 discloses a massaging vibrator gives instantaneous shocks to desired body parts of a person.

### SUMMARY OF THE DISCLOSURE

The invention is defined in the independent claim, with optional features being defined in the dependent claims. In an embodiment, a treatment device includes a housing having a longitudinal axis extending between a proximal end and a distal end, a motor disposed within the housing adjacent the proximal end and having a rotatable motor output shaft; a driveshaft operatively coupled to the motor, the driveshaft having a cam follower and a compression spring disposed about at least a portion of the driveshaft; a helical cam spaced away from the motor output shaft and operatively coupled thereto via a coupler, the coupler being rigidly coupled to the motor output shaft adjacent the proximal end and extending distally thereof to be at least partially disposed about the helical cam, the coupler extending over the cam follower and terminating distal to the cam follower, the cam follower being movable relative to the coupler; a striking element disposed within the housing and operatively driven by the motor to move along the longitudinal axis, a tip disposed adjacent the distal end, and a nose cone disposed about at least a portion of the tip, the tip being moveable within the nose cone, wherein movement of the striking element results in contact with the tip to create an acoustic or shock wave of between 10 and 20 Hertz.

In some examples, a treatment device includes a housing having a longitudinal axis extending between a proximal end and a distal end, a first element disposed at the distal end of the housing, a motor disposed within the housing, a second element operatively coupled to and driven by the motor, and an intermediate element disposed between the first element and the second element, the intermediate element being configured to move between the first element and the second element, and to contact at least one of the first element and the second element.

In at least some examples, a treatment device includes a housing having a longitudinal axis extending between a proximal end and a distal end, a striking element disposed within the housing and moveable along the longitudinal axis, a tip disposed adjacent the distal end, and a position indicator for alerting the user to a proper location on the body to be treated.

In at least some examples, a treatment device includes a housing having a longitudinal axis extending between a proximal end and a distal end, a striking element disposed within the housing and moveable along the longitudinal axis, a tip disposed adjacent the distal end, a processor and a memory.

In at least some examples, a treatment device includes a housing having a longitudinal axis extending between a proximal end and a distal end, a striking element disposed within the housing and moveable along the longitudinal axis, a tip disposed adjacent the distal end, and a speed indicator.

In some examples, a treatment device includes a housing having a longitudinal axis extending between a proximal end and a distal end, a striking element disposed within the housing and moveable along the longitudinal axis, a tip disposed adjacent the distal end, the tip having a flange, and a sensor disposed adjacent the flange of the tip. [Oil] In some examples, a method of treating tissue includes providing a treatment device including a housing having a longitudinal axis extending between a proximal end and a distal end, a striking element disposed within the housing and moveable along the longitudinal axis, a tip disposed adjacent the distal end, the tip having a flange, and a sensor disposed adjacent the flange of the tip, and gathering data on forces between the tip and the tissue over time via the sensor.

### BRIEF DESCRIPTION OF THE DRAWINGS

Various embodiments of the presently disclosed treatment devices are disclosed herein with reference to the drawings, wherein:
FIG. 1A shows a sectional side view of one embodiment of the disclosure wherein the driveshaft is in contact with the tip;
FIG. IB shows a sectional side view of one embodiment of the disclosure wherein the driveshaft is in the rearmost position, on a toe of a cam;
FIG. 2A is a close-up sectional side view of one embodiment of the disclosure illustrating the driveshaft in the forwardmost position and a tip in the forewardmost position;
FIG. 2B is a close-up sectional side view of one embodiment of the disclosure illustrating a driveshaft in the rearmost position and a tip in the resting position;
FIG. 3A is a sectional side view of one embodiment of the disclosure incorporating a transfer slug, and a driveshaft in the forwardmost position of travel;
FIG. 3B is a sectional side view of one embodiment of the disclosure incorporating a transfer slug, and a driveshaft in the rearmost position of travel;
FIG. 4A is a close-up sectional side view of one embodiment of the disclosure incorporating a transfer slug, and a driveshaft in the forwardmost position of travel;
FIG. 4B is a close-up section view of one embodiment of the disclosure incorporating a transfer slug, and a driveshaft in the rearmost position of travel.
FIG. 5 shows a schematic plan view of one embodiment of the disclosure;
FIG. 6 is a schematic translucent plan view of one embodiment of the disclosure showing major components used to create and administer low frequency shock waves;
FIG. 7 is a schematic side sectional view of one embodiment of the disclosure;
FIG. 8 is a schematic closeup sectional side view of one embodiment of the disclosure;
FIG. 9 shows a sectional view of one embodiment of the disclosure illustrating the relative position of major components;
FIG. 10 shows a close-up sectional view of one embodiment of the disclosure with the tip pressure sensor located behind the tip flange;
FIG. 11 shows a transparent plan view of one embodiment of the disclosure illustrating the printed circuit board and annunciator LEDs; and
FIGS. 12-13 show three-colored annunciator lights and a representative output graph of a force detection device integrated into the tip of the subject disclosure.

Various embodiments of the present disclosure will now be described with reference to the appended drawings. It is to be appreciated that these drawings depict only some embodiments of the disclosure and are therefore not to be considered limiting of its scope.

### DETAILED DESCRIPTION OF THE DRAWINGS

Despite the various improvements that have been made to acoustic wave treatment devices, conventional devices suffer from some shortcomings.

There therefore is a need for further improvements to the devices, systems, and methods of manufacturing and using acoustic wave treatment devices. Among other advantages, the present disclosure may address one or more of these needs.

As used herein, the term "proximal," when used in connection with a component of a treatment device, refers to the end of the component farthest from the treatment area, whereas the term "distal," when used in connection with a component of a treatment, refers to the end of the component closest to the treatment area.

Likewise, the terms "trailing" and "leading" are to be taken as relative to the operator of the treatment device. "Trailing" is to be understood as relatively closer to the operator, and "leading" is to be understood as relatively farther away from the operator or closer to the target site of treatment.

In conjunction with the included drawings, this detailed description is intended to impart an understanding of the teachings herein and not to define their metes and bounds. One particular implementation, illustrating aspects of the present teaching, is presented in detail below. Some of the many possible variations and versions are also described.

### 1. ENERGY TRANSFERANCE

Generally, mechanical, electro-mechanical, electronic, electro-hydraulic and pneumatic mechanisms may be used to generate an acoustic wave from a device used for extracorporeal acoustic wave treatments. Each of them involves the rapid acceleration of a projectile from an initial state of rest to a maximum velocity at which point it strikes a target whereby an inelastic transfer occurs of the kinetic energy in the accelerated projectile to the target. Since the target is captive and physically constrained, it cannot be displaced but instead generates an acoustic wave. This acoustic wave may then be transferred to any elastic medium including human tissue. If the target is the tip of the extracorporeal acoustic wave treatment device, and the tip is placed in contact with human tissue, the acoustic signal is transferred to the human tissue. In this manner, the acoustic signal energy, or shock wave, is transferred to the human tissue of the subject thereby effecting beneficial medical treatment.

Certain devices employ costly, fragile, and complicated means of accelerating the projectile which strikes the target and generates the acoustic wave used in treatment. In one example, an electro-mechanical device is used to accelerate the projectile. This electro-mechanical means of accelerating the projectile is intrinsically inexpensive, simple, and robust thereby enabling an inexpensive yet effective extracorporeal acoustic wave treatment device to be introduced into the consumer marketplace at a price point affordable by virtually anyone, making much needed treatments much more widely available than they are at present.

In some examples, energy is instantaneously transferred from an external source to the projectile causing the projectile to accelerate rapidly, strike the target, and return to the initial starting position. Conversely, the instant disclosure relates to a device where a projectile is a shaft which moves in a reciprocating motion by means of a helical cam which bears on a transverse cam follower mechanism attached to the shaft. As the shaft is displaced progressively in the direction opposite the target, the shaft compresses a compression spring, thereby storing energy. The helical cam is rotated by an inexpensive DC motor thereby simply, inexpensively, and robustly converting rotational motion into reciprocating motion. Because the helical cam provides for a gradual progressive compression of the spring, it is possible to store a significant amount of energy utilizing a small, inexpensive motor. Once the cam follower reaches the apex or toe of the cam and drops off, the shaft is accelerated rapidly by virtue of the compressed spring rapidly decompressing. By this means, non-linear reciprocating motion is achieved with a simple, inexpensive, robust mechanism.

In some examples, the driveshaft is accelerated at a high velocity towards the tip or target in order to collide and generate the desired acoustic signal. Without being bound by any particular theory, it is believed that the nature of the mechanism dictates that maximum energy transfer occurs if the collision between the shaft and the tip occurs almost immediately upon release of the stored energy of the compression spring decompressing. As a result of this, the shaft may be subject to significant mechanical interference with the tip, assuming the tip is mechanically constrained and unable to move. This interference may create an inefficient energy transfer between shaft and tip/target resulting in an ineffectual acoustic signal and a failure to generate the desired energy signature from which medical benefit is derived. Moreover, this mechanical interference may place unnecessary and potentially damaging stresses on the mechanical components of the device. Additionally, the total travel distance of the shaft may be constrained by the drop-off height of the helical cam-meaning the cam follower freefalls off the cam toe until it strikes the cam heel. At this point any remaining kinetic energy may be transferred to the helical cam rather than to the tip. For these reasons, alternative configurations may utilize an intermediate member or slug to transfer the kinetic energy from the shaft to the tip while eliminating the disadvantageous problems of mechanical interference between shaft and tip.

The disclosed configurations permit a simple, inexpensive, robust, home use solution which permit self-applied low intensity acoustic wave treatment for various parts of the user's body which would be optimal for the application.

Low intensity acoustic or shock wave generation and transfer means embodying the principles of this disclosure solve the problems of a simple, inexpensive, and robust, home use solution which permits self-applied low intensity acoustic or shock wave treatment for various parts of the user's body. The several embodiments of the disclosure employ designs, materials, and manufacturing methods which are inexpensive and consistent with current manufacturing practices. The functionality, size, cost, simplicity, ease of use, reliability and robustness of the proposed configurations are all advantageous.

Implementations following the principles of this disclosure allow the advantageous modality of a simple, inexpensive, and robust home-use solution which permits self-applied low intensity acoustic or shock wave treatment for various parts of the user's body which would be optimal for the application.

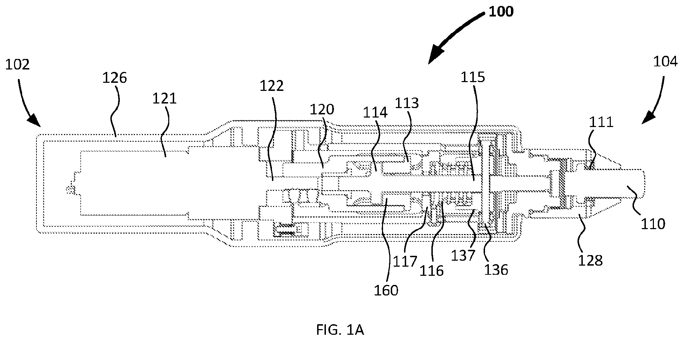

FIG. 1A shows a sectional view of one embodiment of treatment device 100. Treatment device 100 extends between a proximal end 102 and a distal end 104, and includes a housing 126 in the shape of a generally elongated cylinder. Housing 126 may be easily and conveniently grasped in the user's hand in such a manner as to advantageously permit the user to accurately place tip 110 on the desired area of the body to apply treatment. The instant configurations allow energy generated within the device to be transferred to an acoustic wave emanating from tip 110.

Driveshaft 115 is accelerated towards tip 110 for purposes of colliding inelastically and transferring its kinetic energy. Specifically, motor 121 having a motor output shaft 122 is rigidly coupled by means of shaft coupler 120 to helical cam 113. Cam follower 114 may be integral with driveshaft 115 and may be forced into intimate contact with the surface of helical cam 113 via compression spring 116. As helical cam 113 is rotated due to the rotation of motor 121, cam follower 114 is drawn rearwards towards motor 121 by virtue of displacement by the ramp profile of helical cam 113, said displacement causing compression spring 116 to compress. Compression spring 116 may be at its proximal end constrained by spring base plate 117 and at its distal end constrained by spring cap 137 which is rigidly affixed to driveshaft 115.

As driveshaft 115 moves progressively rearwards (i.e., translates along the longitudinal axis toward proximal end 102), towards motor 121 by virtue of the ramping action of helical cam 113 displacing cam follower 114, compression spring 116 becomes more and more compressed.

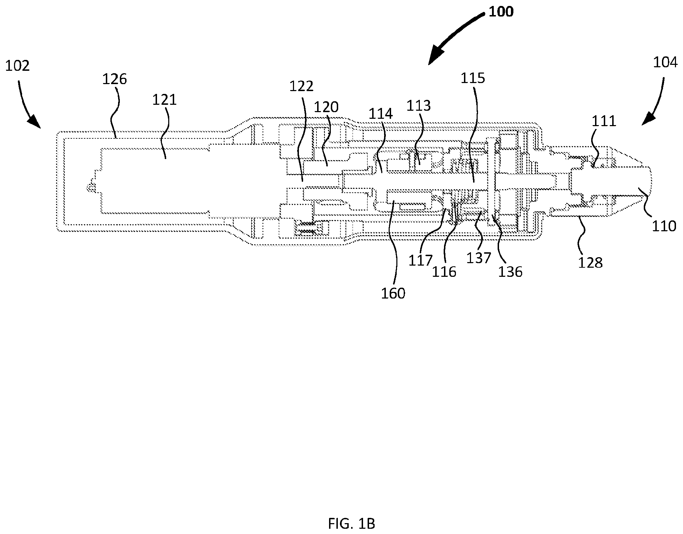

Referring now to FIG. 1B which is a sectional view of one embodiment of the disclosure, it can be seen that driveshaft 115 is in the rearmost position on the toe 160 of helical cam 113. It is at this precise moment during the rotation of helical cam 113 when driveshaft 115 has reached maximum rearward displacement and compression spring 116 is under maximum compressive load. After further rotation of helical cam 113, cam follower 114 falls off of the toe 160 of helical cam 113 permitting compression spring 116 to rapidly decompress, thereby imparting kinetic energy to driveshaft 115, accelerating it rapidly towards the distal end of the device, and toward tip 110.

In this configuration, kinetic energy of driveshaft 115 is imparted efficiently to tip 110. With many low intensity acoustic or shock wave devices, tip 110 is physically constrained within nose cone 128, inhibiting it from motion along its longitudinal axis. Given that the impact of driveshaft 115 with tip 110 occurs while driveshaft 115 is at maximal acceleration, and therefore still traveling longitudinally after being accelerated by compression spring 116, were it to contact tip 110 and be forced to stop traveling, there m indeed be a transfer of kinetic energy, but much of the energy may be absorbed by nose cone 128 and housing 126 rather than being transferred entirely to tip 110 and thereby to the user's treatment area. In order to avoid such undesirable energy transfer to nose cone 128 and housing 126, tip 110 is disposed at least partially within the nose cone 128 and permitted longitudinal freedom of motion.

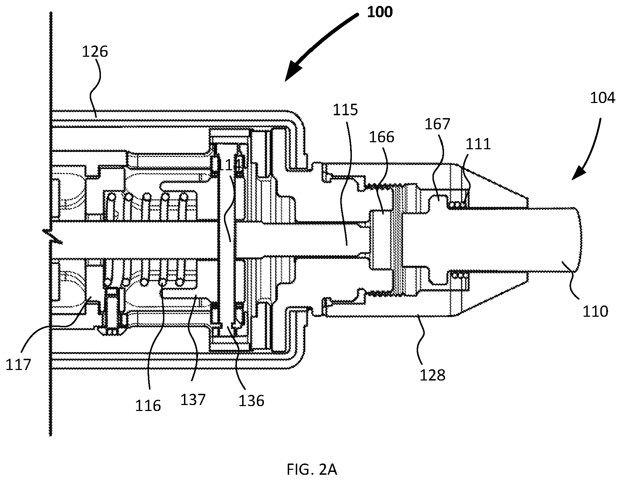

Referring now to FIG. 2A, which is a close-up sectional view of the tip and nose cone assembly of one embodiment of the disclosure, the arrangement of the major components may be plainly seen and the operation of the device may be readily understood. In this view, driveshaft 115 is in the fully forward position, maximally displaced towards tip 110. Also, in this view, it may be seen that tip 110 is in the fully forward displaced position after having been displaced by contact with driveshaft 115. Also visible is return spring 111 which is a compression spring which bears against nose cone 128 and annular ring 167 integrally formed with tip 110. The purpose of return spring 111 is to permit tip 110 to accelerate and displace forward, yet return tip 110 back to its proximal-most resting position in anticipation of the next collision with driveshaft 115. Return spring 111 may have a predetermined spring constant so as to provide a slight force that is still sufficient to permit the return of tip 110 back to its resting position while countering the force of tip 110's acceleration as little as possible. In this configuration, tip 110 may freely accelerate forward after being struck by the accelerating driveshaft 115 as a result of the impact which occurs when the limit of forward travel of the forwardmost face of driveshaft 115 interferes with the resting rearwardmost face of tip 110 when in the resting or rearwardmost position. In this view, it may be seen that return spring 111 is fully compressed as a result of the forward movement of tip 110, said stored energy in return spring 111 now ready to return tip 110 rearwards back to its resting position, ready for the next impact from driveshaft 115. In this manner, tip 110 is properly positioned for impact with driveshaft 115 each time driveshaft 115 accelerates forward rapidly upon cam follower 114 falling off of cam toe 160, thereby permitting compression spring 116 to decompress and accelerate driveshaft 115.

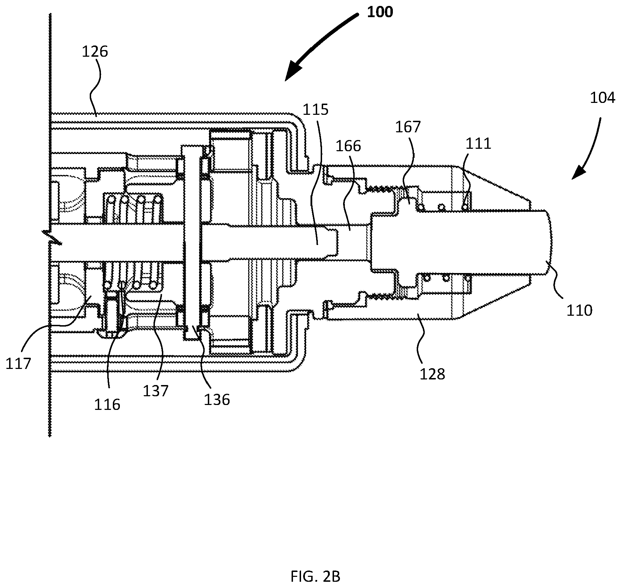

Referring now to FIG. 2B, a close-up sectional view of the tip and nose cone assembly of one embodiment of the disclosure is shown. Driveshaft 115 is in the rearwardmost (i.e., proximal-most) position, tip 110 is in the resting or rearwardmost (i.e., proximal-most) position, and return spring 111 is in its relaxed and uncompressed state. In this view, it may also be seen that compression spring 116 is in its maximally compressed state, with maximum stored energy, ready to be released. In this condition, an air gap 166 is shown between driveshaft 115 and tip 110. Air gap 166 is at its maximum length, permitting ample distance for driveshaft 115 to accelerate due to the decompression of compression spring 116 once cam follower 114 falls off of cam toe 160, thereby releasing the stored energy in compression spring 116. At the moment that driveshaft 115 reaches its maximum acceleration, the forwardmost tip of driveshaft 115 strikes the rearwardmost face of tip 110, causing tip 110 to accelerate forward as a result of the inelastic collision between driveshaft 115 and tip 110. In this manner, the stored energy of compression spring 116 as translated into the acceleration of driveshaft 115, and is transferred to tip 110 to the treatment area of the patient.

This configuration contemplates two elements to create an acoustic wave, namely a striking element (e.g., driveshaft) and a moveable element (e.g., tip). In an alternate embodiment of the disclosure, the transfer of stored energy in compression spring 116 is transferred to tip 110 in other ways using, for example, additional components.

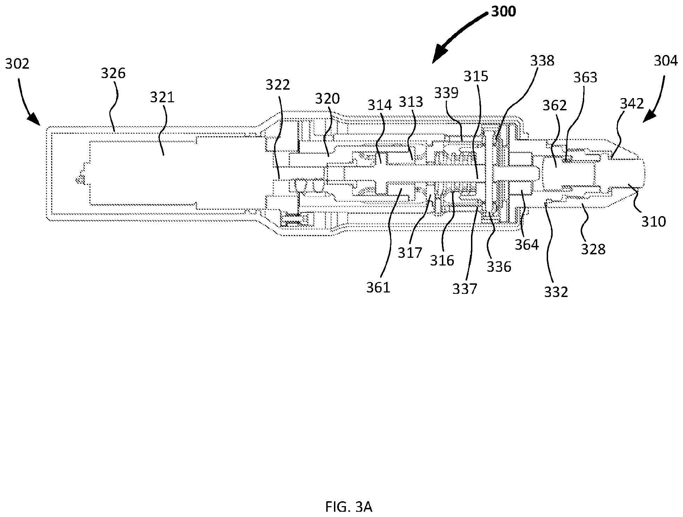

Referring now to FIG. 3A, which is a sectional view of the tip and nose cone assembly of an alternative embodiment of the disclosure, the arrangement of the major components of device 300 may be plainly seen and the operation of the device may be readily understood. In this view, driveshaft 315 is in the fully forward (distalmost) position, on the heel 361 of helical cam 313 maximally displaced toward the proximal end of device 300. Also, in this view, it may be seen that there is an additional intermediate component, transfer slug 362 having a flared end, which resides in the space between the distal end of driveshaft 315 and the proximal end of tip 310. In this view, transfer slug 362 is in the forwardmost position and its distal end is in intimate contact with the rearwardmost face of tip 310. Transfer slug return spring 363 is in the fully compressed condition, ready to release the stored energy and return transfer slug 362 back to its starting or rearwardmost position. In this arrangement, tip 310 is loosely constrained against longitudinal movement, its annular ring 367 trapped between the distal face of transfer housing 332 and proximal face of nose cone 328 with o-rings 342 in the interstitial space permitting tip 310 to vibrate freely as a result of the collision impact and energy transfer of transfer slug 362.

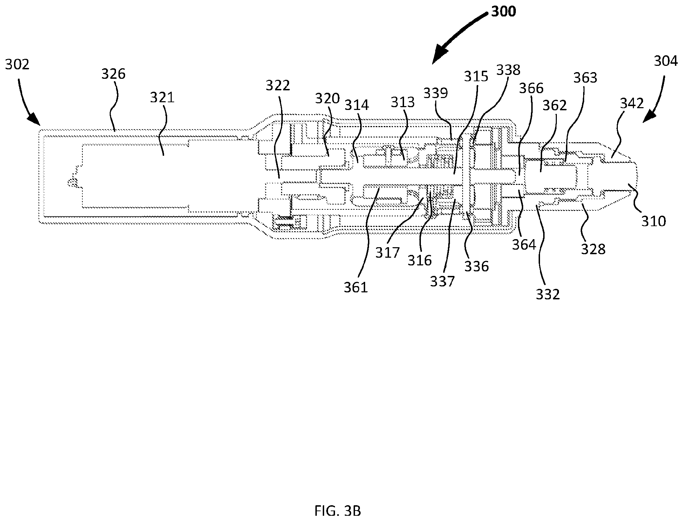

Referring now to FIG. 3B which is a section view of the tip and nose cone assembly of an alternative embodiment of the disclosure, the arrangement of the major components may be plainly seen and the operation of the device may be readily understood. In this view, driveshaft 315 is in the fully rearward position, on the toe 360 of helical cam 313. Transfer slug 362 is in the rearwardmost position and transfer slug return spring 363 is in the uncompressed or relaxed state.

The manner of operation of this mechanism is as follows. Still referring to FIG. 3B, compression spring 316 is in its fully compressed state, storing the maximum possible energy as a result of cam follower 314 having progressively compressed compression spring 316 as a result of riding along the inclined face of helical cam 313 to the point of maximum displacement, cam toe 360. At this instant, transfer slug 362 is resting in its rearwardmost position with an air gap 366 between the proximal tip of driveshaft 315 and the distal face of transfer slug 362, as well as an air gap 366 between the proximal face of transfer slug 362 and the distal face of tip 310.

After this point, as cam follower 314 slips off of cam toe 360 and the stored energy of compression spring 316 is instantaneously released, driveshaft 315 is rapidly accelerated longitudinally towards transfer slug 362. There is an ensuing inelastic collision between the proximal tip of driveshaft 315 and the distal face of transfer slug 362 during which the kinetic energy of driveshaft 315 is transferred to transfer slug 362, thereby causing it to rapidly accelerate longitudinally towards the proximal end of device 300.

As transfer slug 362 accelerates longitudinally, it closes air gap 366 and collides inelastically with the distal face of tip 310, thereby transferring its kinetic energy. As tip 310 is constrained against longitudinal motion, the kinetic energy causes tip 310 to vibrate, thereby propagating the acoustic wave energy into any material with which it comes into contact, in this instance preferably the soft tissue or target treatment area of the patient or user.

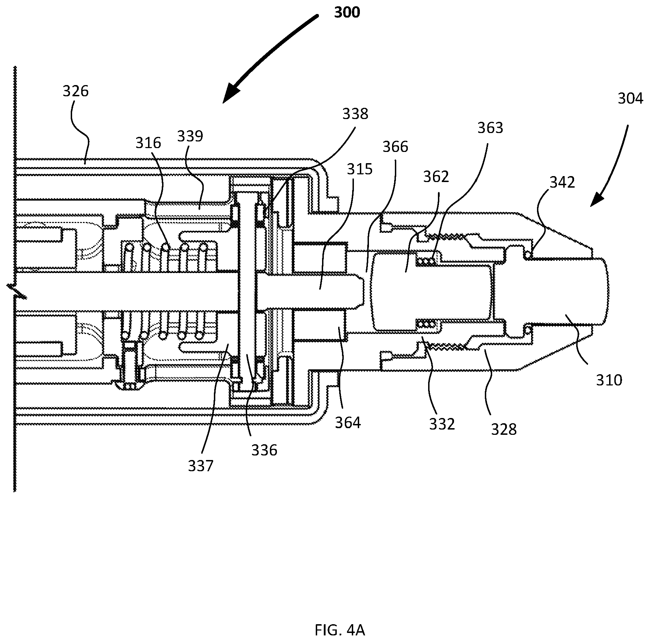

FIG. 4A is a close-up sectional view of this alternate embodiment of the disclosure. It may be seen how transfer slug 362 makes intimate contact with tip 310 when accelerated longitudinally as a result of impact from driveshaft 315, and how said inelastic collision results in the transfer of kinetic energy from the rapidly accelerating transfer slug 362 to captive tip 310, thereby causing it to ring or resonate, and thereby transferring the energy to the target treatment area. In this manner, the reciprocating motion of driveshaft 315 may be accommodated without encumbrance or mechanical interference while no reciprocating motion is communicated to tip 310, thereby creating a purely vibrational acoustic wave energy transfer which is more familiar to users of existing low intensity acoustic or shock wave devices.

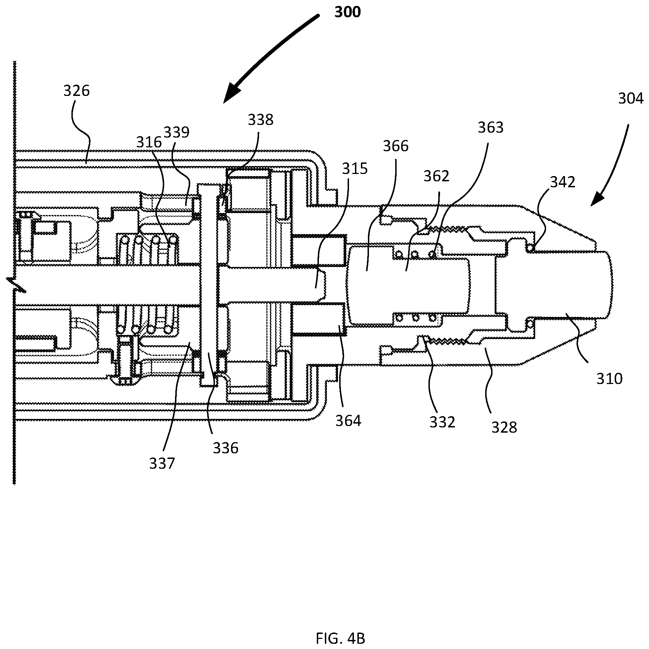

Referring now to FIG. 4B, a close-up sectional view of this alternate embodiment of the disclosure is shown. This configuration aims to reduce lost energy (e.g., energy lost through friction) and to provide the maximal transfer of energy from compression spring 316 through driveshaft 315 to transfer slug 362 to tip 310 and ultimately to the target treatment area of the patient. These efforts include linear bushings 364 or in an alternate embodiment, linear bearings, which guide driveshaft 315 in its longitudinal reciprocating motion. The device also includes close tolerance and accurate coaxial bores and outside diameters of transfer housing 332 and transfer slug 362, and even the anti-rotation axle 336 with roller bearings 338, which reside within and travel along guide track 339 to resist the torqueing moment of cam follower 314 as it tracks along the helical cam 313 ramp, thereby affording a low friction, non-binding reciprocating motion of driveshaft 315.

Thus, the present disclosure includes a variety of mechanisms to efficiently transfer energy generated by the device to provide inexpensive electric motor to compress a compression spring, from a decompressing compression spring to the tip of a device which administers low intensity acoustic or shock waves to targeted areas of the user's body for treatment of soft tissue damage, cellulite reduction, or erectile dysfunction which is a safe, inexpensive, reliable, robust, and which would be optimal for the application.

### 2. TREATMENT ASSISTANCE FEATURES

Conventional devices generally have a form factor that is intended for a professional medical provider to administer treatment to a third-party subject or patient and as such is not ergonomically well suited for an individual to self-administer treatment. Additionally, there are no displays and controls that are readily visible or accessible to an individual self-administering treatment. Conventional devices also do not provide any information regarding proper positioning of the device during treatment to assist an untrained user in the proper positioning of the device during use.

In contrast, the present disclosure contemplates devices capable of providing information regarding proper rate of travel of the device during treatment to assist an untrained user in the proper movement of the device during use. The present devices and methods also provide information regarding the number of passes completed at any given time of the device during treatment to assist an untrained user in the proper duration of treatment of the device during use. The present device and methods also monitor and/or limit the operating time of the device during treatment to assist an untrained user in avoiding over-treatment of the device. For example, the proposed methods and devices may include monitoring and/or limiting of the total number of operating cycles of the device to assist an untrained user in understanding the operational service life, and need for periodic maintenance of the device.

The present devices and methods address problems with the limited viewing angle of the information display of the device which is detrimental to a self-administering user who must hold the device in a variety of positions and orientations during use, while still needing to see the displayed information during use. The also address problems associated with significant decibel levels of sound produced by the device during use which alerts others in proximity to the user that the device is in use and thereby prevents the discreet use of the device such as may be desired by a user from time to time.

The devices and methods also may provide communication and/or messaging capability, which may be useful to communicate additional information to the user including tutorials, patient tracking, system updates, and marketing, sales, and/or promotion messaging. The device also provides means of remote viewing or imaging which may be useful to communicate to a self-administering user the precise location of the tip during use. The proposed solutions also provide the ability to pay for the device and/or operation of the device on a per-treatment basis.

While there are presently a number of proposed solutions to the problem of devices which utilize extracorporeal shock therapy for purposes of treating soft tissue damage, cellulite reduction, or erectile dysfunction, none has been conceived or implemented to permit a simple, inexpensive, robust, home use solution which permit self-applied low intensity shock wave treatment for various parts of the user's body with a form factor, display, information, guidance, tutorials and training, marketing communication and purchase opportunities, viewing angle, timers, annunciators, sound attenuation, and payment options which provide an untrained amateur user with all tools and guidance necessary to properly and safely self-administer treatments which would be optimal for the application.

Low intensity shock wave generation and transfer means embodying the principles of this disclosure solve the problems of a simple, inexpensive, robust, home use solution which permit self-applied low frequency shock wave treatment for various parts of the user's body with a form factor, display, information, guidance, viewing angle, timers, annunciators, and sound attenuation which provide an untrained amateur user with all tools and guidance necessary to properly and safely self-administer treatments for various parts of the user's body as well as including the capability to display and communicate tutorials, patient tracking, system updates, and marketing, sales, and promotion messaging. The several embodiments of the disclosure employ designs, materials, and manufacturing methods which are inexpensive and consistent with current manufacturing practices. The functionality, size, cost, simplicity, ease of use, reliability and robustness of the proposed designs are all advantageous.

Implementations following the principles of this disclosure allow the advantageous modality of a simple, inexpensive, robust, home use solution which permit self-applied low frequency shock wave treatment for various parts of the user's body with a form factor, display, information, guidance, viewing angle, timers, annunciators, and sound attenuation which provide an untrained amateur user with all tools and guidance necessary to effectively and safely self-administer treatments shock wave treatment for various parts of the user's body and have the capability of delivering tutorials, patient tracking, system updates, and marketing, sales, and/or promotion messaging which would be optimal for the application.

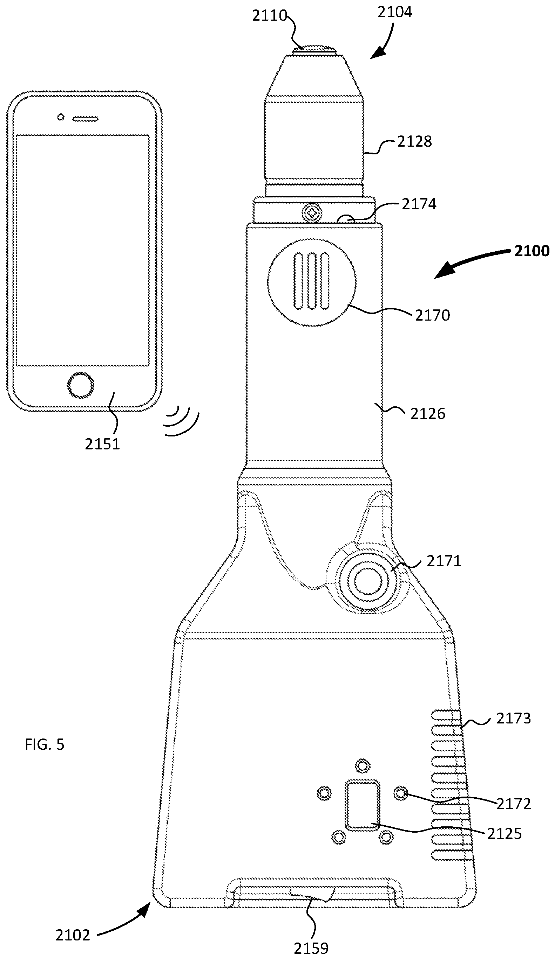

FIG. 5 shows a plan view of one embodiment of the device 2100 extending between proximal end 2102 and distal end 2104. Device 2100 includes a housing 2126 in the form of an elongated, generally cylindrical body which is easily and conveniently grasped in the user's hand in such a manner as to advantageously permit the user to accurately place tip 2110 on the desired area of the body to apply treatment. Display 2125 is advantageously positioned so as to permit an unobstructed line of site for the viewer from the proximal end 2102 toward the distal end 2104 during most normal usage. End cap or nose cone 2128 is easily accessible and removably attached to housing 2126 by any of a variety of conventional means including an internal screw thread, an interrupted thread, a snap lock or any of a variety of mechanical fasteners, so as to facilitate the simple installation, removal, replacement, or switching of tip 2110 as required for the selected treatment. In one embodiment, nose cone 2128 is secured to housing 2126 by an interrupted thread which requires no tools and only a partial rotation to remove and replace. Bar graph segments 2173 are readily viewed by the user when the device is positioned and held for use; and annunciator LEDs 2172 are also readily seen during use of device 2100.

Still referring to FIG. 5, smartphone 2151 may be seen to be in proximity to device 2100 and may be in communication with the device 2100. In at least some examples, smartphone 2151 may be wirelessly data connected to device 2100 by means of a communication module, for example Bluetooth or any of a variety of other wireless data connection means and protocols, for purposes of wirelessly communicating with device 2100. It is through such communication of smartphone 2151 with device 2100, more specifically with printed circuit board 2134 and the electronic components situated thereon including microprocessors and other semi-conductors and a memory, that a bi-lateral communication means may be established between device 2100 and smartphone 2151. This bi-lateral communication means may be advantageously utilized for communication, display, control, monitoring, and/or marketing functions.

Given that smartphone 2151 is able to communicate wirelessly with device 2100, it may be utilized to perform several functions which are advantageous for the user. First, it may serve as the control panel and information display for device 2100. This is advantageous for several reasons. First, smartphone 2151 likely has a touch screen and large color display which enable a very high-quality graphical user interface which may variably and preferentially display information and/or controls as needed. Smartphone 2151 may also advantageously be placed in a location which is optimal for the user to see displayed information and interact with controls to turn the device 2100 on and off, or stop and start operation of the device 2100.

Given that smartphone 2151 is capable of internet enabled communication, it may be used to provide any of the following: tutorial and coaching information to the user including 'how-to' videos prior to or during use, usage tips; health, diet, exercise, and lifestyle tips to maximize results; and sales and marketing opportunities to purchase consumables, upgrade or purchase additional units, purchase spare parts; and social links to connect the user with user groups, community bulletin boards, and other social media resources including potentially dating sites, clubs, organizations, and groups. Smartphone 2151 may also be utilized to keep track of treatments, results, user progress, and provide reminders about upcoming treatments or recommended ancillary treatments or products. Further, smartphone 2151 may provide user access to frequently asked question resources and potentially also to live chat or human operators for additional assistance.

In some examples, device 2100 may be deployed on parts of the user's body which are difficult to see with direct line of site for example when the device is being used to reduce cellulite on the buttocks or back of the thigh, yet which are important to visually monitor during use, device 2100 may be equipped with a camera 2174 which may be of a closed circuit, webcam or any of a wide variety of other cameras well known in the art, so located proximal to the nose cone 2128 of device 2100 by which means it provides a field of view including tip 2110 of device 2100 and the treatment area of the user, which can communicate the image data with smartphone 2151 which displays it, thereby enabling the user to see where tip 2110 of device 2100 is being placed during use.

In some examples, smartphone 2151 is capable of internet enabled communication and e-commerce functions with a remote server, and it may serve as the secure communication, monitoring, and payment portal through which a fundamentally new model of service may be enabled-a 'pay per pulse' or 'pay as you go' model wherein device 2100 is sold at a very low cost, or given at no cost to the end user, but will not operate until activated. Activation is accomplished by means of an application or 'app' which is downloaded and installed on the user's smartphone 2151, and which by means of secure and/or encrypted wireless communication with device 2100 permits the user to purchase by means of e-commerce or any of a wide range of well-known transaction means activation permission for the device 2100 which activation permission is quantified by treatment duration or intensity, number of pulses of operation, or duration of use, or by any other delimiting factor deemed necessary or desirable. Once a purchase has been made in-app, the user's smartphone 2151 communicates securely with device 2100 by means of the aforementioned secure wireless communications link and authorizes device 2100 to operate at the user's discretion to the limit of the purchased activation permission. By this means, two of the greatest obstacles to the commercial sale of this device are overcome: first, the initial purchase price, which may be dramatically lower than would be the case for a device which did not require purchase of activation permission which is essentially a means of spreading the total cost of purchase and operation over time; and second, the concern that the device will no perform as promised or will not for any of a variety of reasons be acceptable or desirable for the purchaser and in the event that the device was purchased at full price without the requirement of activation permissions, there is a far greater risk of financial loss for the purchaser if they elect not to utilize the device after purchase. The activation permission model essentially distributes the risk far more favorably for the purchaser.

Still referring to FIG. 5, it may be seen how several of the control and display features including alphanumeric display 2125, annunciator LEDs 2172, and LED bar graph 2173 advantageously communicate critical operational status and tutorial information to the user as follows:

Upon power up by means of on/off switch 2159, alphanumeric display 2125, annunciator LEDs 2172, and LED bar graph 2173 are illuminated. This indicates that device 2100 has been energized and is ready for use and operation. Given that device 2100 is intended for use by a user who most likely has no special training or medical knowledge, one of the principal challenges, which has been met by one of the inventive steps disclosed herein, is the means by which an untrained user may safely and effectively self-administer treatment with the device. The first challenge for the untrained user is to understand which line of travel to follow with tip 2110 of device 2100. In the instance that device 2100 is being used to treat erectile dysfunction, the treatment protocol specifies 5 lines of travel longitudinally along the user's penis. These lines of travel are along the top centerline, and along the upper and lower sides of the penis as most readily communicated by calling them out as positions on a clock face-for example 12 o'clock is the top centerline, 2 o'clock is the upper left side line of travel, etc. To this end, annunciator LEDs 2172 are so arranged and oriented on device 2100 as to provide a simple and unambiguous guide to the user indicating which line of travel is to be treated, which has already been treated, and which has not yet received treatment. To facilitate the user knowing which line of travel is being called for at each stage of the treatment, annunciator LEDs 2172 may exhibit one of 3 states when device 2100 is energized. In the unilluminated state, the LED remains off and no color is displayed. This indicates that the corresponding line of travel has not yet been treated. If annunciator LED 2172 is illuminated green, this indicates that this particular line of travel is currently being treated, indicating that this is the line of travel upon which tip 2110 of device 2100 is to be placed. Once treatment along this line of travel has been completed, annunciator LED 2172 changes to red illumination color. This indicates that treatment for this line of travel has been completed. To further illustrate the point, upon initial power-up of device 2100, all annunciator LEDs 2172 are off, except the line of travel which is to receive the first treatment, which will be illuminated green. Upon completion of 4 lines of treatment, 4 annunciator LEDs 2172 will be illuminated red, and the annunciator LED 2172 indicating the final line of treatment will be illuminated green.

In order that the user has time to place tip 2110 of device 2100 on the appropriate line of travel and to prepare to administer the treatment, device 2100 is energized upon switching on of on/off switch 2159 coupled to a power source (e.g., battery or plug), but it does not begin operating or generating energy waves for treatment. Once device 2100 is in position, the user presses momentary start button 2171 to activate the device 2100. Upon activation, device 2100 begins emitting sound waves from tip 2110, for treating the target treatment area.

Still referring to FIG. 5, it may be plainly seen how LED bar graph 2173 is advantageously positioned to be visible to the user during operation of device 2100. The function of LED bar graph 2173 is to provide the user with visual guidance for the rate of travel of tip 2110 of device 2100 across the treatment area. Bar graph 2173 eliminates the need for the device to be operated by skilled, trained medical professionals. Instead, bar graph 2173 allows for operation by an unskilled user, and reduce or eliminate the risk of physical injury as a result of improper use. The single greatest risk to the user is the administration of an excessive number of sound wave pulses to any one area of soft tissue. Low intensity shock wave devices are intended to be in constant motion along a treatment path of travel, rather than remaining in one location for a period of time. A second risk to the user is that of reduced efficacy of treatment because the rate of travel of tip 2110 is too fast. To safeguard against both eventualities and to make device 2100 more safely and effectively operated and self-deployed by an untrained user, the inventive step of a pacing LED bar graph 2173 has been incorporated in device 2100 to provide an analogue visual reference guide for the rate of travel of tip 2110 along the designated treatment path. The 10 emissive elements of LED bar graph 2173 begin all illuminated upon initial energizing of device 2100. One LED however, either in position 1 or position 10 will not be steadily illuminated, but will instead be flashing. By this means, the user will know upon which end of the line of travel to place tip 2110 and thereby upon which end of the line of travel to begin treatment.

As treatment begins, the user moves tip 2110 of device 2100 along the line of treatment. The rate of travel is likely something an untrained user has no awareness of and conventional means of quantifying a rate of travel are difficult to comprehend and difficult to translate into operation for an untrained, unskilled user; yet proper rate of travel of tip 2110 of device 2100 is crucial to the safety and efficacy of the device. To this end, LED bar graph 2173 begins decrementing immediately upon treatment initiation once momentary start button 2171 has been depressed. Decrementing is accomplished by turning off each successive LED in LED bar graph 2173 after one tenth of the treatment time duration for a single line of travel has elapsed. For example, if the duration of treatment for one pass on one line of treatment travel is 10 seconds, one LED of LED bar graph 2173 will extinguish illumination each second, in succession. By this means, the user will be able to visually equate the percentage of the line of treatment which has been treated as well as the percentage of the line of treatment remaining to be treated with the percentage of LEDs extinguished and illuminated respectively. For example, the user will readily comprehend that when the first 5 LEDs are extinguished, and the next 5 LEDs remain illuminated, the tip 2110 of device 2100 should be halfway along the line of treatment, such that the line of treatment will be completed and the tip 2110 of device 2100 will be at the terminus point of the line of treatment concurrent with the extinguishment of the final LED, number 10. By this means, the user is able to perform a safe and effective treatment based on a proper rate of travel along each line of treatment.

Upon completion of one pass of device 2100 along a line of travel of treatment, LED bar graph 2173 once again illuminates all emissive elements, with all elements illuminated steadily, except for the last LED which blinks, indicating that this is the end of the line of travel at which the next treatment pass will begin. The LEDs will now begin successively extinguishing, once again providing a proportional visual analog for the user to properly gauge the position and rate of travel of device 2100 at any point in the treatment cycle.

Still referring to FIG. 5, we now discuss the function of alphanumeric display 2125. Upon initial energization of device 2100, alphanumeric display 2125 will display the total number of passes required for the first line of treatment travel. In the event that the treatment protocol calls for 10 passes on each line of treatment, alphanumeric display 2125 will initially display the number 10. Upon activation of device 2100 by pressing momentary start button 2171, and the initiation of one pass along the line of treatment travel by the user, alphanumeric display 2125 will continue to display the number of passes for the current line of travel, until such time as the device reaches the terminus point of the line of travel as indicated by the extinguishment of all segments of LED bar graph 2173, at which point the alphanumeric display 2125 will decrement one count, in this case now display "9" indicating that 9 passes remain on this treatment cycle for this line of travel. Alphanumeric display 2125 will continue to decrement until the full count of passes per the treatment protocol has been executed. At this time, device 2100 will be de-activated, meaning it ceases producing sonic waves, and concurrently the annunciator LED 2172 for the just competed line of travel will change in illumination from green to red and the next indicated line of travel annunciator LED 2172 will illuminate green indicating that the user should reposition tip 2110 of device 2100 on the indicated line of travel and prepare to initiate another phase of the treatment. By this means, even the unskilled user may perform a safe and effective treatment with proper positioning of lines of travel, proper rate of travel of device 2100 and proper counts for passes along each line of travel. It is anticipated that each of the aforementioned variables including duration of treatment per pass along a line of travel, number and location of lines of travel, rate of travel, and number of passes along each line of travel may be altered or varied depending on the treatment type and patient needs, and may be altered interactively in the case that device 2100 is coupled to the internet by means of smartphone or some other means of connectivity well known in the art.

Upon completion of a complete course of treatment comprising the prescribed number of passes along each line of treatment travel for all prescribed lines of treatment travel, the device will go into a sleep mode wherein it will not operate for a prescribed period of time, perhaps 36 hours or 72 hours or 96 hours. The purpose of this timeout function is to prevent over-use by an overly enthusiastic user lacking medical knowledge or training, thereby not understanding that excessive use will not only not yield greater results buy may actually be harmful or dangerous. By this means, yet another inventive step, the untrained user is once again limited to a prescribed course of treatment which is safe and effective.

Alphanumeric display 2125 may be utilized to display the number of hours remaining in the time out mode until operation of the device may resume.

A further software resident operating limitation may dictate total operational life of the device wherein after a certain total cumulative number of pulses the unit enters a 'sunset' mode where it will no longer operate. Conversely, it may simply give a visual indication on the alphanumeric display 2125 or some such other display that the unit has reached the end of its design life and requires service or replacement. The purpose of this feature is to safeguard against the continued usage of the device beyond the design life, after which the energy signature may be compromised due to the excessive wear or failure of high stress components within the device responsible for generating the shock wave.

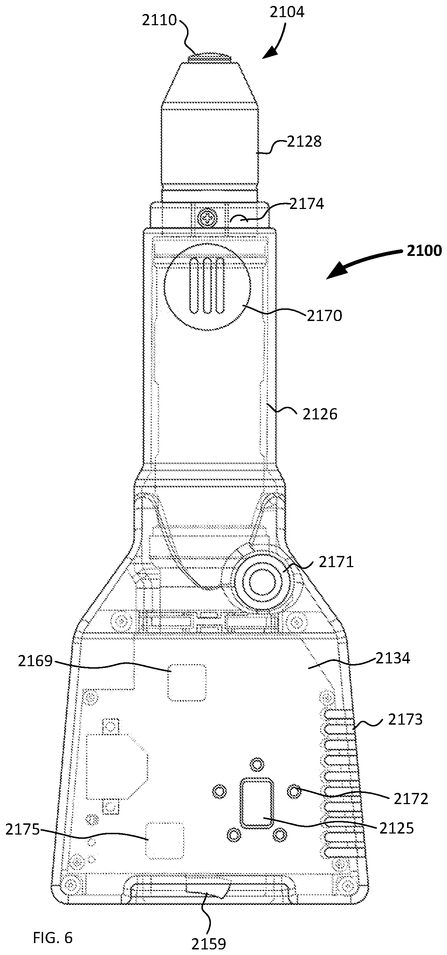

Referring now to FIG. 6 which is a translucent plan view of one embodiment of the disclosure, the arrangement of certain major components may be plainly seen including the placement and location of printed circuit board 2134 advantageously positioned to support the aforementioned controls and displays, and also incorporating blue tooth communication integrated circuit 2175 and audio processor/amplifier 2169.

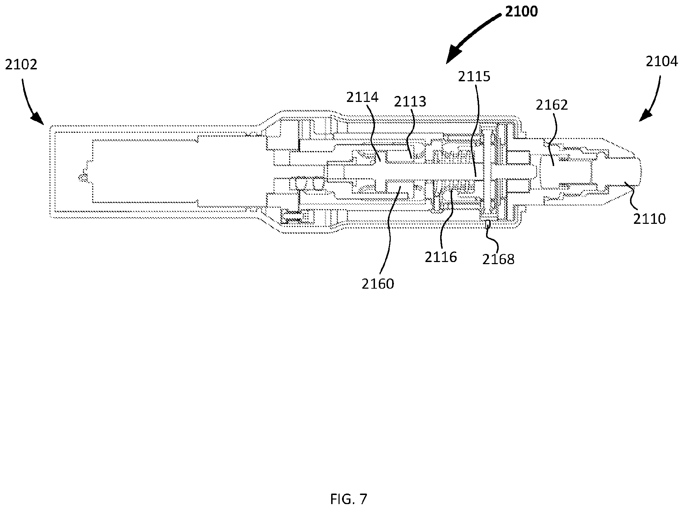

Referring now to FIG. 7 which is side section view of one embodiment of the device, it may be plainly seen how major components of the assembly are arranged and interact with one another, particularly driveshaft 2115, transfer slug 2162, and tip 2110. In this view it may be seen how compression spring 2116 is compressed by cam follower 2114 riding the ramps of helical cam 2113 until such time as cam follower 2114 falls off of cam toe 2160 thereby precipitously releasing the stored energy in compression spring 2116 which causes driveshaft 2115 to rapidly accelerate towards transfer slug 2162. Further, it may be understood how driveshaft 2115 collides with transfer slug 2162 with great velocity and kinetic energy, and as a result of this collision transfer slug 2162 collides with tip 2110 with similarly great velocity and kinetic energy. These repeated collisions which in the preferred embodiment of device 2100 occur 15 times per second create a high decibel sound which may be problematic for users for several reasons. First, the sheer decibel level and frequency of the sound may be painful to listen to and potentially harmful to the hearing of anyone in close proximity, thereby requiring hearing protection during operation of the device 2100; and second, in some instances a user desires discretion during use of the device 2100 so as not to alert others in close proximity to the fact of their utilization of the device. For both of these reasons it may be desirable to significantly reduce the decibel level of the operation noise created by the device 2100.

While certain materials and design practices well known in the art may be employed to attenuate these sounds passively, it may be desirable to further attenuate sound levels beyond that which may be accomplished by such means. To this end, it may be desirable to employ a noise mitigation technique known as active noise cancelling.

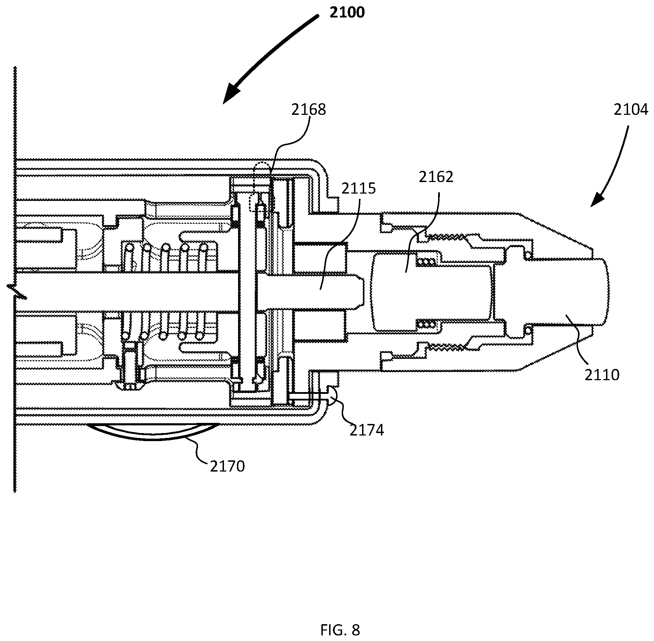

Referring now to FIG. 8 which is a close up side section view of the proximal end of the preferred embodiment of the device, it may be more plainly seen how driveshaft 2115, transfer slug 2162 and tip 2110 interact with one another and collide inelastically to transfer energy from the initial storage source of compression spring 2116 to driveshaft 2115, to transfer slug 2162, to tip 2110 and thereby to the treatment area of the user. Active noise cancellation which is well known in the art has never been applied to a low intensity shock wave device. Such devices are particularly well suited to sound attenuation by active noise cancellation given that active noise cancellation works best in an environment where the objectionable sounds are consistent, fixed, predictable, and occurring at regular intervals-essentially precisely the operating conditions of device 2100.

Still referring to FIG. 8, it may be seen how microphone 2168 is so positioned and located as to be able to monitor the sound energy created by the collision of driveshaft 2115 with transfer slug 2162, and with transfer slug 2162 and tip 2110. The monitored signal is transmitted to audio processor/amplifier 2169 on printed circuit board 2134, both visible in FIG. 6. Audio processor/amplifier 2169 analyzes the signal received from microphone 2168 and generates an identical audio signal which is phase shifted one half phase in order to effectively cancel the sound emanating from the device. The amplified phase shifted signal is transmitted to speaker 2170 which may be a standard cone and voice coil, piezo electric or any of a variety of other speaker types well known in the art and which may actually be multiple speakers strategically located so as to optimally cancel the offensive original generated noise from device 2100, said speaker emitting a phase shifted mimic of the original detected sound which will in large part cancel the overall sound of the device, and by this means render the device much quieter for safe, discreet operation.

It may be seen in these several embodiments of the disclosure that the disclosure overcomes the deficiencies of all previous attempts at solving the problem of device which administers low intensity shock waves to targeted areas of the user's body for treatment of soft tissue damage, cellulite reduction, or erectile dysfunction which is a safe, inexpensive, self-applied, home use solution which does not require a second person to operate, significant medical or anatomical knowledge, special training, and which provide for tutorials, patient tracking, system updates, and marketed, sales, and promotion capabilities, which would be optimal for the application.

In broad embodiment, the present disclosure is a device which administers low frequency shock waves to targeted areas of the user's body for treatment of soft tissue damage, cellulite reduction, or erectile dysfunction which is a safe, inexpensive, self-applied, home use solution which does not require a second person to operate, significant medical or anatomical knowledge, special training, and which provide for tutorials, patient tracking, system updates, and marketing, sales, and promotion capabilities, and reduced noise, which would be optimal for the application.

### 3. COMMUNICATION FEATURES

Generally, mechanical, electro-mechanical, electronic, electro-hydraulic and pneumatic mechanisms may be used to generate an acoustic wave from a device used for extracorporeal acoustic wave treatments. Each of them involves the rapid acceleration of a projectile from an initial state of rest to a maximum velocity at which point it strikes a target whereby an inelastic transfer occurs of the kinetic energy in the accelerated projectile to the target. Since the target is captive and physically constrained, it cannot be displaced but instead generates an acoustic wave. This acoustic wave may then be transferred to any adjacent medium including human tissue. If the target is the tip of the extracorporeal acoustic wave treatment device, and the tip is placed in contact with human tissue, the acoustic signal is transferred to the human tissue. In this manner, the acoustic signal energy, or acoustic wave, is transferred to the human tissue of the subject thereby effecting beneficial medical treatment.

Conventional devices are generally operated by a trained medical professional for treatment of a third-party subject. The efficacy of the treatment is, in part, determined by the pressure with which the tip of the treatment device is held against the target tissue. If the tip pressure is too light, energy transfer is inefficient and treatment benefit will be diminished. If the tip pressure is too great, there is a risk of injury to the tissue. Thus, there may be an optimal range of tip pressure for proper transfer of the acoustic wave energy from the tip of the device to the target tissue.

Given that the device is usually somewhat heavy, is constantly in motion over the target tissue, and is vibrating as a result of the high energy acoustic wave generation mechanism within, it is somewhat difficult for the user to monitor or gauge the tip pressure against their tissue simply by feel. With all known prior acoustic wave treatment devices intended to be operated by professionals and deployed on a third party, it could be relied upon that the trained operator had sufficient knowledge and skill to maintain appropriate tip pressure during treatment. In some cases, costly professional devices monitor and display tip pressure in units which are meaningless to an untrained user, and without value attribution with respect to proper, target, desired, insufficient, or potentially harmful levels and thereby require expert knowledge and interpretation. But in the case of self-treatment by an untrained user, absent a quantifiable measure of tip pressure and clear interpretation of proper and acceptable ranges of tip pressure, it is likely that proper tip pressure will not be easily or consistently maintained throughout a treatment, thereby undermining efficacy of the treatment. It is therefore deemed desirable for there to be an objective means by which tip pressure may be continuously monitored and annunciated to the user to facilitate maintaining proper tip pressure throughout the entirety of the treatment. The means by which this optimal tip pressure is detected and annunciated to the user is the subject of this patent.

While there are presently a number of proposed solutions to the problem of monitoring and annunciating contact pressure of the tip of an acoustic wave device to be applied to an area of the body for soft tissue damage, cellulite reduction, or erectile dysfunction treatment, none has been conceived or implemented to permit a simple, inexpensive, robust, self-use solution which permit self-applied acoustic wave treatment for various parts of the user's body while maintaining proper tip pressure which would be optimal for the application.

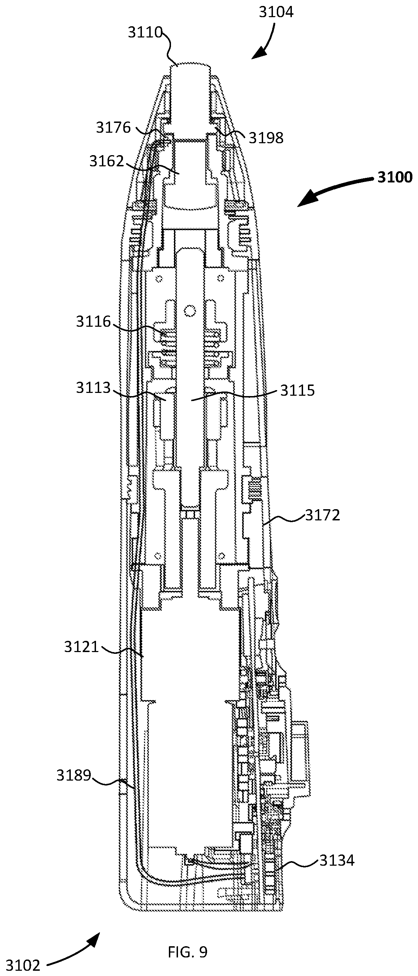

FIG. 9 shows a side sectional view of one embodiment of the device 3100 extending between a proximal end 3102 and a distal end 3104, wherein the general arrangement of major components may be plainly seen including motor 3121 which rotates helical cam 3113, which causes driveshaft 3115 to compress compression spring 3116, wherein the stored energy of compressed compression spring 3116 is periodically released causing driveshaft 3115 to accelerate rapidly forwards and collide with transfer slug 3162 which is then accelerated forward to collide with tip 3110 thereby imparting an acoustic wave to the user.

Still referring to FIG. 9, it may be seen how force detector 3176 is adjacent to, and located behind or rearwards of, the flange 3198 on tip 3110. In the preferred embodiment, force detector 3176 is a piezoelectric load cell, but in other embodiments it may be any of a wide range of force detecting devices or technologies well known in the art including accelerometers or electro-mechanical detectors. The output of force detector 3176 is communicated via signal wires 3189 to printed circuit board (PCB) assembly 3134 where the signals are processed to be displayed to the user.

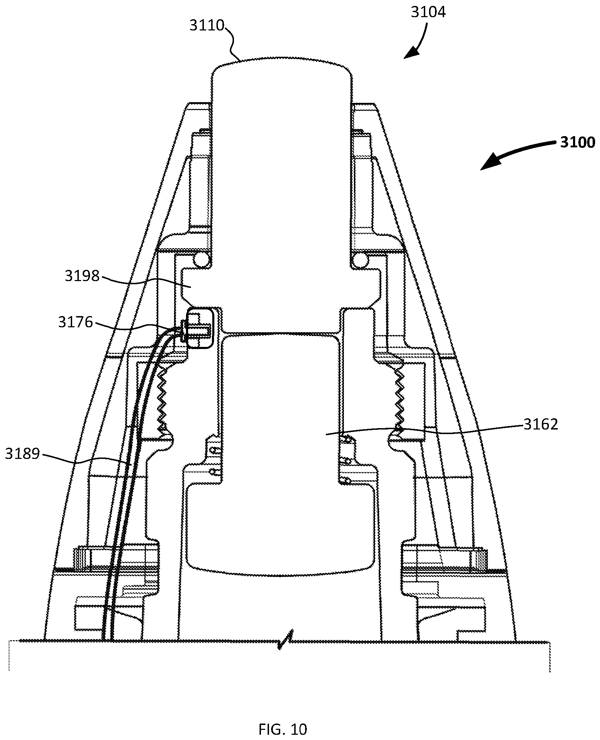

Referring now to FIG. 10 which is a close-up sectional view of the tip and nose cone assembly of one embodiment of the disclosure, the arrangement of the force detector 3176 relative to tip 3110 may be plainly seen and the operation of the device may be readily understood. In this view, it may be seen how the force detecting face of force detector 3176 rests against the rearward face of tip flange 3198 wherein the axis of detection of force detector 3176 is aligned with the axis of motion of tip 3110 such that when tip 3110 is pressed against the user's flesh, this force will be applied to force detector 3176, which then generates an electrical signal which is communicated to PCB for display to the user.

Still referring to FIG. 10, it may be seen how when transfer slug 3162 is accelerated forward to collide with tip 3110, the resulting collision with tip 3110 produces a tip force in the opposite direction of that generated when tip 3110 is pressed against the user's flesh. In the first case, the tip force is generated in a direction away from tip force detector 3176 while in the second case, the tip force generated is towards tip force detector 3176. Given that these forces are occurring with regular frequency corresponding to the pulse per second operational rate of the device, and given that they occur in opposing directions, the resulting force measurement diagram will approximate a sine wave.

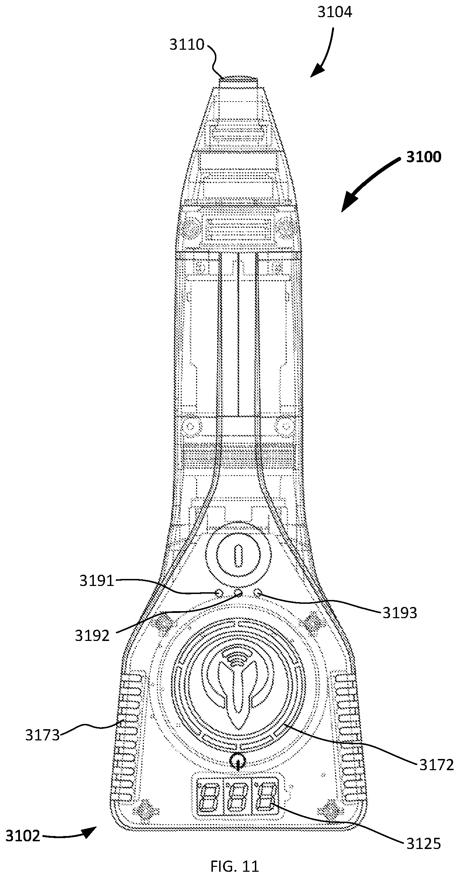

Referring now to FIG. 11 which is a transparent plan view of the subject device, it may be seen how annunciator LEDs 3191, 3192, and 3193 are mounted on PCB assembly 3134 such that they are visible to the user during operation of the device 3100 within view of other critical display annunciators including alphanumeric display 3125, bar graph displays 3173 and line of travel displays 3172.

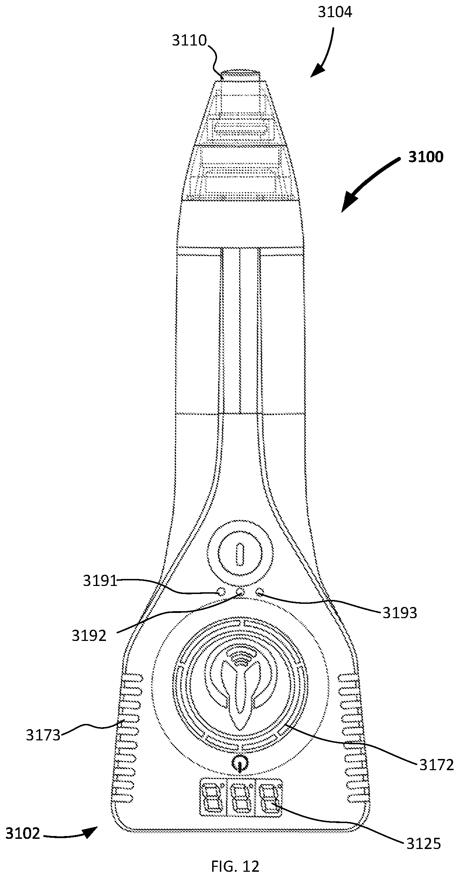

Referring now to FIGS. 12-13 which is a plan view of the device of the subject disclosure and a graphical representation of the tip force experienced by tip force detector 3176 during operation, it may be more plainly seen how the opposing forces generated by the impact of transfer slug 3162 with tip 3110, and the contact of tip 3110 with user's flesh will be measured by tip force detector 3176.

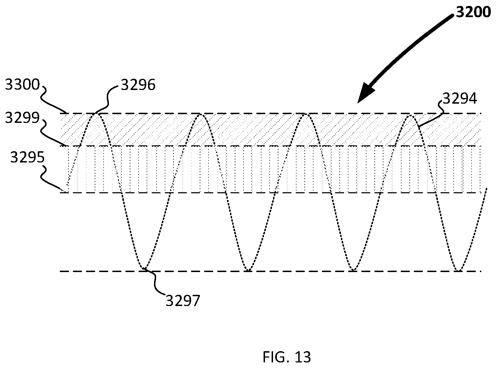

Still referring to FIG. 13, a graphic representation 3200 shows a plurality of thresholds. For example, a horizontal line 3295 represents zero force detection by tip force detector 3176. Line 3295 serves as a baseline reference that represents a condition when tip 3110 is not biased from resting position by any external forces-not pressed against user's flesh, and not struck by transfer slug 3162. The sinusoidal wave 3294 represents the tip force readings measured by tip force detector 3176 during operation of the device.

The tip force detector 3176 measures the maximum amplitude of the sine wave in each direction, negative (e.g., away from the distal end) and positive (e.g., toward the distal end). In FIG. 13, the maxima of waveform 3294 are indicated by a maximum amplitude in a positive direction indicated by peak 3296, and a maximum amplitude of the negative direction tip force as indicated by lower peak 3297. In some examples, lower peak 3297 may not be used, but in another embodiment of the disclosure this data may be used to monitor and quantify the tip force which is of particular value and interest in embodiments of the device where tip force is user adjustable or by some other means variable, such as remotely programmable by a physician or third party.

Still referring to FIG. 13, it may be seen that the amplitude of the positive going tip force is indicated by upper peak 3296. This portion of waveform 3294 is the target, or desired tip force for optimal device performance and energy transfer to the user's tissue. Line 3300 represents a maximum threshold, or a positive going tip force which is excessive and potentially harmful to the user, and line 3299 represents a minimum threshold. Three tip force regions are formed with maximum threshold 3300 and minimum threshold 3299: a region below line 3299, a region between line 3299 and 3300, and a region above line 3300, and these regions help determined significant values which the present disclosure quantifies and communicates to the user. Given that tip force detector 3176 is subject to a continuously variable sinusoidal force profile by virtue of periodic impacts from transfer slug 3162, it may be clearly seen how for purposes of monitoring tip force, only the maximum positive going amplitude of tip force waveform 3294 is monitored (e.g., peak 3296).

Still referring to FIGS. 12-13, three annunciator lights 3191-3193 may be used to communicate this data to the user. Colored LEDs are used in the preferred embodiment of the disclosure, but in other embodiments they may be other emissive devices or may be integrated into a display screen and displayed as a bar graph, pie-chart, or some other analog or representative symbol, or voice prompt or sound signal, or haptic signal such as a vibration felt by the user's hand communicate the tip force to the user by indicating which zone, as identified in the preceding paragraph, tip force corresponds to. The annunciator lights are color coded using a 'traffic light' color coding convention well understood by most adults wherein yellow indicated caution, green indicates 'go' and red indicates 'stop.' The color-coding convention used by the device is similar in the yellow indicates inadequate tip force, green indicates proper tip force and red indicates excessive tip force.

If tip force is below the minimum threshold level indicated by line 3299 in FIG. 13, for illustrative purposes below 10 pounds per square inch (psi), the yellow annunciator 3191 illuminates indicating to the user that inadequate tip force is detected and they must press down on device 3100 harder to increase tip force against the tissue. Once detected tip force is within the target range for illustrative purposes between 10 psi and 20 psi, green annunciator 3192 illuminates and continues to illuminate while the force within the target region. If tip force exceeds the maximum limit of, for illustrative purposes 20 psi, red annunciator 3193 illuminates. It must be noted that the stated pressure values are illustrative only, and actual pressures will be determined based on treatment type and tip characteristics including surface area, material, and profile.

By this means, the user has a simple, color or symbol-based, easy to follow, easy to interpret means of understanding how much tip force they are applying and adjusting it accordingly if it is too little or too much simply by pressing down more or less during operation. This completely eliminates the need to understand or interpret data or force measurements which requires prior knowledge and training as to target values and which is distracting during a self-administered treatment as multiple other displays and actions must be observed and coordinated. Maintaining proper tip force becomes as simple as pressing down on the tip until the green light illuminates and adjusting tip force during use to maintain illumination of the green light. If the green light extinguishes and yellow light illuminates, the user knows to apply more pressure. Conversely, if the green light extinguishes and the red light illuminates, the user knows to back off of tip pressure until the green light illuminates once again. This insures a simple, accurate, constant feedback to maintain ideal tip pressure during use of the device 3100. In an alternative embodiment there are only 2 lights-a green light and a red light. There would be no illumination until tip pressure reaches the minimum acceptable threshold at which time a green light would illuminate. If the maximum acceptable threshold is exceeded, the green light would extinguish and the red light would illuminate. These red and green emissive elements may be combined in the same light source such as a bi-color LED. In yet another embodiment, device operation could be inhibited until such time as minimum acceptable tip pressure is achieved, meaning acoustic waves would not be generated and relevant timer and travel rate displays would not be activated until such time as sufficient tip pressure was present.

In broad embodiment, the present disclosure is a simple, inexpensive, and robust, home use solution which permits maintaining proper tip pressure during treatment of self-applied low frequency shock wave treatment for various parts of the user's body by an untrained user. The several embodiments of the disclosure employ designs, materials, and manufacturing methods which are inexpensive and consistent with current manufacturing practices. The functionality, size, cost, simplicity, ease of use, reliability and robustness of the proposed designs are all advantageous.

While the foregoing written description of the disclosure enables one of ordinary skill to make and use what is considered presently to be the best mode thereof, those of ordinary skill will understand and appreciate the existence of variations, combinations, and equivalents of the specific embodiment, method, and examples herein. The disclosure should therefore not be limited by the above described embodiment, method, and examples, but by all embodiments and methods within the scope of the invention as defined in the appended claims.

## Claims

1. A treatment device (100) comprising:
a housing (126) having a longitudinal axis extending between a proximal end (102) and a distal end (104);
a motor (121) disposed within the housing (126) adjacent the proximal end (102) and having a rotatable motor output shaft (122);
a driveshaft (115) operatively coupled to the motor (121), the driveshaft (115) having a cam follower (114) and a compression spring disposed about at least a portion of the driveshaft (115);
a helical cam (113) spaced away from the motor output shaft (122) and operatively coupled thereto via a coupler (120), the coupler (120) being rigidly coupled to the motor output shaft (122) adjacent the proximal end (102) and extending distally thereof to be at least partially disposed about the helical cam (113), the coupler (120) extending over the cam follower (114) and terminating distal to the cam follower (114), the cam follower (114) being movable relative to the coupler (120);
a striking element disposed within the housing (126) and operatively driven by the motor (121) to move along the longitudinal axis;
a tip (110) disposed adjacent the distal end (104); and
a nose cone (128) disposed about at least a portion of the tip (110), the tip (110) being moveable within the nose cone (128) along the longitudinal axis;
wherein movement of the striking element results in contact with the tip (110) to create an acoustic or shock wave of between 10 and 20 Hertz.

2. The treatment device of claim 1,
wherein the helical cam is disposed adjacent the compression spring, the helical cam having a first flat end and a second end having at least one discontinuity.

3. The treatment device of claim 2, wherein the striking element is the driveshaft.

4. The treatment device of claim 2, wherein the driveshaft actuates the striking element.

5. The treatment device of claim 1, further comprising a return spring at least partially disposed about the tip.

6. The treatment device of claim 1, further comprising a return spring at least partially disposed within the nose cone.

7. The treatment device of claim 1, further comprising an annular ring coupled to the tip.

8. The treatment device of claim 1, further comprising an annular ring integrally formed with the tip.

9. The treatment device of claim 8, further comprising a return spring seated about the tip and abutting the annular ring.

10. The treatment device of claim 9, further comprising a variable air gap disposed between the tip and the striking element, the variable air gap having a length along the longitudinal axis that at least partially depends on whether the return spring is compressed.

11. The treatment device of claim 1, wherein the coupler covers but does not contact the cam follower.

## Patentansprüche

1. Behandlungsvorrichtung (100), die umfasst:
ein Gehäuse (126) mit einer Längsachse, die sich zwischen einem proximalen Ende (102) und einem distalen Ende (104) erstreckt;
einen Motor (121), der im Gehäuse (126) neben dem proximalen Ende (102) angeordnet ist und eine drehbare Motorausgangswelle (122) aufweist;
eine Antriebswelle (115), die mit dem Motor (121) betriebsmäßig verbunden ist, wobei die Antriebswelle (115) einen Nockenstößel (114) und eine Druckfeder aufweist, die um mindestens einen Teil der Antriebswelle (115) angeordnet ist;
eine spiralförmige Nocke (113), die von der Motorausgangswelle (122) beabstandet ist und über eine Kupplung (120) betriebsmäßig mit dieser verbunden ist, wobei die Kupplung (120) neben dem proximalen Ende (102) starr mit der Motorausgangswelle (122) verbunden ist und sich distal davon erstreckt, um zumindest teilweise um die spiralförmige Nocke (113) angeordnet zu sein, wobei sich die Kupplung (120) über den Nockenstößel (114) erstreckt und distal zum Nockenstößel (114) endet, wobei der Nockenstößel (114) relativ zur Kupplung (120) beweglich ist;
ein Schlagelement, das im Gehäuse (126) angeordnet ist und vom Motor (121) betriebsmäßig angetrieben wird, um sich entlang der Längsachse zu bewegen;
eine Spitze (110), die neben dem distalen Ende (104) angeordnet ist; und
einen Nasenkegel (128), der um zumindest einen Teil der Spitze (110) angeordnet ist, wobei die Spitze (110) innerhalb des Nasenkegels (128) entlang der Längsachse beweglich ist;
wobei die Bewegung des Schlagelements zum Kontakt mit der Spitze (110) führt, wodurch eine Schall- oder Stoßwelle zwischen 10 und 20 Hertz erzeugt wird.

2. Behandlungsvorrichtung nach Anspruch 1, wobei die spiralförmige Nocke neben der Druckfeder angeordnet ist, wobei die spiralförmige Nocke ein erstes flaches Ende und ein zweites Ende mit mindestens einer Diskontinuität aufweist.

3. Behandlungsvorrichtung nach Anspruch 2, wobei das Schlagelement die Antriebswelle ist.

4. Behandlungsvorrichtung nach Anspruch 2, wobei die Antriebswelle das Schlagelement betätigt.

5. Behandlungsvorrichtung nach Anspruch 1, die ferner eine Rückholfeder umfasst, die zumindest teilweise um die Spitze angeordnet ist.

6. Behandlungsvorrichtung nach Anspruch 1, die ferner eine Rückholfeder umfasst, die zumindest teilweise innerhalb des Nasenkegels angeordnet ist.

7. Behandlungsvorrichtung nach Anspruch 1, die ferner einen ringförmigen Ring umfasst, der mit der Spitze verbunden ist.

8. Behandlungsvorrichtung nach Anspruch 1, die ferner einen ringförmigen Ring umfasst, der integral mit der Spitze ausgebildet ist.

9. Behandlungsvorrichtung nach Anspruch 8, die ferner eine Rückholfeder umfasst, die um die Spitze sitzt und am ringförmigen Ring anliegt.

10. Behandlungsvorrichtung nach Anspruch 9, die ferner einen variablen Luftspalt, der zwischen der Spitze und dem Schlagelement angeordnet ist, umfasst, wobei der variable Luftspalt eine Länge entlang der Längsachse aufweist, die zumindest teilweise davon abhängt, ob die Rückholfeder zusammengedrückt ist.

11. Behandlungsvorrichtung nach Anspruch 1, wobei die Kupplung den Nockenstößel abdeckt, ihn jedoch nicht berührt.

## Revendications

1. Dispositif de traitement (100) comprenant :
un boîtier (126) présentant un axe longitudinal s'étendant entre une extrémité proximale (102) et une extrémité distale (104) ;
un moteur (121) disposé à l'intérieur du boîtier (126) adjacent à l'extrémité proximale (102) et présentant un arbre de sortie de moteur rotatif (122) ;
un arbre d'entraînement (115) couplé fonctionnellement au moteur (121), l'arbre d'entraînement (115) présentant un suiveur de came (114) et un ressort de compression disposé autour d'au moins une partie de l'arbre d'entraînement (115) ;
une came hélicoïdale (113) à l'écart de l'arbre de sortie de moteur (122) et couplée fonctionnellement à celui-ci via un coupleur (120), le coupleur (120) étant couplé rigidement à l'arbre de sortie de moteur (122) adjacent à l'extrémité proximale (102) et s'étendant à distance de celui-ci de façon à être disposé au moins partiellement autour de la came hélicoïdale (113), le coupleur (120) s'étendant par-dessus le suiveur de came (114) et se terminant à distance du suiveur de came (114), le suiveur de came (114) étant mobile par rapport au coupleur (120) ;
un élément de frappe disposé à l'intérieur du boîtier (126) et entraîné fonctionnellement par le moteur (121) pour se déplacer le long de l'axe longitudinal ;
une pointe (110) disposée adjacente à l'extrémité distale (104) ; et
un cône de nez (128) disposé autour d'au moins une partie de la pointe (110), la pointe (110) étant mobile à l'intérieur du cône de nez (128) le long de l'axe longitudinal ;
dans lequel le mouvement de l'élément de frappe aboutit au contact avec la pointe (110) pour créer une onde acoustique ou de choc comprise entre 10 et 20 Hertz.

2. Dispositif de traitement selon la revendication 1, dans lequel la came hélicoïdale est disposée adjacente au ressort de compression, la came hélicoïdale présentant une première extrémité plate et une deuxième extrémité présentant au moins une discontinuité.

3. Dispositif de traitement selon la revendication 2, dans lequel l'élément de frappe est l'arbre d'entraînement.

4. Dispositif de traitement selon la revendication 2, dans lequel l'arbre d'entraînement actionne l'élément de frappe.

5. Dispositif de traitement selon la revendication 1, comprenant en outre un ressort de rappel disposé au moins partiellement autour de la pointe.

6. Dispositif de traitement selon la revendication 1, comprenant en outre un ressort de rappel disposé au moins partiellement à l'intérieur du cône de nez.

7. Dispositif de traitement selon la revendication 1, comprenant en outre une bague annulaire couplée à la pointe.

8. Dispositif de traitement selon la revendication 1, comprenant en outre une bague annulaire formée d'un seul tenant avec la pointe.

9. Dispositif de traitement selon la revendication 8, comprenant en outre un ressort de rappel placé autour de la pointe et venant en butée contre la bague annulaire.

10. Dispositif de traitement selon la revendication 9, comprenant en outre un interstice variable entre la pointe et l'élément de frappe, l'interstice variable présentant une longueur le long de l'axe longitudinal qui dépend au moins partiellement du fait que le ressort de rappel soit comprimé ou non.

11. Dispositif de traitement selon la revendication 1, dans lequel le coupleur recouvre le suiveur de came sans pour autant être en contact avec celui-ci.
